# EUROPEAN PATENT APPLICATION

(11) **EP 1 505 158 A1**
(43) Date of publication of application: **09.02.2005**
(21) Application number: 03425700.6
(22) Date of filing: 29.10.2003
(51) Int. Cl.: C12Q 1/68

(54) **Method and kit for diagnosing the genetic predisposition of sheep to scrapie**

(30) Priority: 07.08.2003 IT MI20031627
(71) Applicant: Nuclear Laser Medicine S.r.l., 20090 Settala (MI) (IT)
(72) Inventor: De Renzio, Pietro, 20129 Milano (IT); Spinelli, Luigi, 22046 Mariano Comense (CO) (IT); Clemenza, Filippo, 10100 Torino (TO) (IT)
(74) Representative: Cattaneo, Elisabetta

(57) **Abstract**

The present invention relates to a method for determining the predisposition of sheep to scrapie by determining the presence or absence of the DNA sequences corresponding to one or more polymorphisms at sites 136, 154 and 171 of the prion protein, comprising the steps of isolating the DNA from uncoagulated blood and amplifying one or more primers in such a way as to generate amplified products complementary to the said polymorphic sites, and is characterized in that the primers generating the amplified products are labelled with one or more labels, and the said labelled amplified products are subsequently hybridized on strips to oligonucleotide probes corresponding to the said polymorphic sites. A kit for applying the method according to the invention is also described.

SEQUENCE LISTING

<110>  NUCLEAR LASER MEDICINE S.r.l.

<120>  Method and kit for diagnosing the genetic predisposition of sheep to
scrapie

<130>  P02207/EP

<140>  EP 03425700.6
<141>  2003-10-29

<150>  MI2003A001627
<151>  2003-08-07

<160>  9

<170>  PatentIn version 3.1

<210>  1
<211>  20
<212>  DNA
<213>  Artificial Sequence

<220>
<223>  description of Artificial Sequence: primer oligonucleotide sequen
       ce
<400>  1
tgcagctgga gcagtggtag                                                20

<210>  2
<211>  22
<212>  DNA
<213>  Artificial Sequence

<220>

<223>  description of Artificial Sequence: primer oligonucleotide sequen
      ce

<400>  2
gtggtgactg tgtgttgctt ga                                              22

<210>  3

<211>  17

<212>  DNA

<213>  Artificial sequence

<220>

<223>  description of Artificial Sequence: probe oligonucleotide sequenc
      e for detecting polymorphism at the 136 codon of gene relating to
       the prion protein in sheep

<400>  3
tggcacttcc cagcatg                                                    17

<210>  4

<211>  17

<212>  DNA

<213>  artificial sequence

<220>

<223>  description of Artificial Sequence: probe oligonucleotide sequenc
      e for detecting polymorphism at the 136 codon of gene relating to
       the prion protein in sheep

<400>  4
tgacacttcc cagcatg                                                    17

<210>  5

<211>  21

<212>  DNA

<213>  artificial sequence

<220>

<223>  description of Artificial Sequence: probe oligonucleotide sequenc
      e for detecting polymorphism at the 154 codon of gene relating to
       the prion protein in sheep

<400>  5
ctatcgtgaa aacatgtacc g                                               21

<210>  6

<211>  22

<212>  DNA

<213>  artificial sequence


<220>

<223>  description of Artificial Sequence: probe oligonucleotide sequenc
       e for detecting polymorphism at the 154 codon of gene relating to
        the prion protein in sheep

<400>  6
actatcatga aaacatgtac cg                                                22

<210>  7

<211>  22

<212>  DNA

<213>  artificial sequence


<220>

<223>  description of Artificial Sequence: probe oligonucleotide sequenc
       e for detecting polymorphism at the 171 codon of gene relating to
        the prion protein in sheep

<400>  7
ccagtggatc agtatagtaa cc                                                22

<210>  8

<211>  21

<212>  DNA

<213>  artificial sequence


<220>

<223>  description of Artificial Sequence: probe oligonucleotide sequenc
       e for detecting polymorphism at the 171 codon of gene relating to
        the prion protein in sheep

<400>  8
ccagtggatc ggtatagtaa c                                                 21

<210>  9

<211>  22

<212>  DNA

<213>  artificial sequence

<220>

<223>  description of Artificial Sequence: probe oligonucleotide sequenc
       e for detecting polymorphism at the 171 codon of gene relating to
        the prion protein in sheep

<400>  9
ccagtggatc attatagtaa cc                                                22

## Description

The present invention relates to a method and kit for diagnosing the genetic predisposition of sheep to scrapie.

Scrapie is a neurodegenerative disease known for about three centuries. It belongs to the family of transmissible spongiform encephalopathies (TSE), also known as prion diseases. They are neurodegenerative diseases which affect the central nervous system of humans and animals, and are characterized by a chronic, slow but progressive development with an inevitably fatal outcome.

It is known that a prion, a protein normally present in the cell membrane of the neurons and therefore present in large numbers in the brain and central nervous system, can undergo a change of conformation which transforms it from a normal prion, "PrP", to a pathological prion, "PrPsc". Because of this conformational change, PrPsc can no longer be attacked by the enzymes which normally degrade proteins, and indeed it has a "dominating" effect on normal prions, transforming them into the pathological PrPsc form.

In this form, it is associated with the infectivity of scrapie and can therefore be a factor for the determination of the disease.

Scrapie is particularly widespread in European flocks and affects adult animals at ages between two and four and a half years, but is only rarely encountered in 18-month-old animals. The incubation period from the moment of infection is particularly long (from two to five years), while the clinical course in sheep ranges from 8 to 24 weeks.

Genetic predisposition to the disease has been determined, with the discovery that there are specific genetic sequences and consequently different genotypes associated with the PrP protein.

It is known, in fact, that some genetic sequences are associated with susceptibility to scrapie.

The relationship between the development of the clinical signs of scrapie in sheep and polymorphisms of the gene relating to the prion protein has been described in the literature. The most important of these polymorphisms appear to be those corresponding to the 136, 154 and 171 codons, in other words the VRQ variant. [O'Doherty E, Averne M and Ennis S, "Prion protein gene polymorphism in pedigree sheep in Ireland", 1: Res Vet Sci 2001 Feb; 70(1): 51-6].

It is known that the polymorphism in the 136 codon consists in the replacement of the alanine (A) amino acid residue (A) with that of valine (V) [A136V], the polymorphism in the 154 codon consists in the replacement of the arginine (R) amino acid with histidine (H) [R154H], and finally the polymorphism in the 171 codon consists in the replacement of glutamine (Q) with either arginine (R) or histidine (H) [Q171R and Q171H].

The document WO01/21835 discloses a method for determining the presence or absence of at least one PrP allele in the polymorphic sites 171, 154 or 136, using the PCR process. In this method, at least one primer which is substantially complementary to a target DNA sequence of the PrP allele is chosen, the target sequence comprising at least one polymorphic site. Specifically, the said at least one primer is substantially complementary to the said target sequence up to the nucleotide located immediately before one of the polymorphic sequences, and there is no bond between the primer and the target sequence starting at said nucleotide located immediately before the polymorphic site. This method provides greater reliability by reducing the number of false positive results. This is because the primer with a sequence perfectly complementary to the DNA sequence including the polymorphic site provides a PCR product, while a primer with at least two non-complementary nucleotides does not produce PCR. Thus there is a greater control over the false positives which sometimes occur even with only one non-complementary nucleotide base.

There are also other known methods of diagnosing scrapie, based on PCR amplification with primers complementary to the sequence in question and detection on gel.

Regardless of the primers used to decrease the false positives, these methods have proved to be unsuitable for detecting low concentrations of DNA.

On the other hand, methods using restriction enzymes are highly expensive at present, as is the method of complete DNA- sequencing, which is also notably time-consuming.

Interest in these methods and kits has also been demonstrated at the European level, with a proposal for legislation to regulate the screening of sheep to permit genetic selection by identification and slaughtering of animals affected by the disease.

Clearly, therefore, an increase in the reliability of the determination of the polymorphic sequences, combined with practicality and ease of use of the method, will make the selection and rearing of scrapie-resistant animals more certain.

The object of the present invention is therefore to provide a method and a kit for determining the predisposition to scrapie.

Another object of the present invention is to provide a fast and practical method for determining susceptibility to scrapie.

The present invention achieves these objects by providing the method indicated in Claim 1 and the kit according to Claim 10. Further advantageous characteristics are indicated in the dependent claims.

The present invention therefore provides a method for determining the predisposition to scrapie by determining the presence or absence of the DNA sequences corresponding to one or more polymorphisms at sites 136, 154 and 171 of the prion protein, comprising the following steps:
a) isolating the DNA from uncoagulated blood;
b) amplifying one or more primers in such a way as to generate amplified products complementary to the said polymorphic sites,
characterized in that the primers generating the amplified products of step b) are labelled with one or more labels, and the said labelled amplified products are subsequently hybridized on strips to oligonucleotide probes corresponding to the said DNA sequences relating to the said polymorphic sites.

The method according to the present invention is described in detail below with reference to an example of embodiment of the invention which is to be interpreted as an example and not as a limitation of the invention, and with reference to the figures, wherein:
Figure 1 shows a reference strip for determining the genotype; and
Figure 2 shows the strips A-J resulting from the use of the kit according to the invention.

The step of isolating the DNA according to the present invention can preferably be carried out by column extraction. The blood sample used can be fresh blood, or blood stored at 4°C or frozen at -20°C. Preferably, the blood used for the DNA extraction is frozen blood.

In an alternative embodiment of the invention, the blood sample can be taken in the form of spots on absorbent paper. In this case, the fresh blood sample is absorbed and dried on absorbent paper at 4°C, after which the absorbent paper slip is dissolved in PBS in a test tube and subjected to vortex agitation, then incubated at ambient temperature. The same test tube is then used in the extraction step.

The primers used in the following invention are preferably
5'-Mar-TgC AgC Tgg AgC AgT ggT Ag, and
5'-Mar-gTg gTg ACT gTg TgT TgC TTg A,
where Mar denotes labelling,
which are added in step b) of the method according to the present invention by means of an amplification mixture preferably also containing a buffer, DNAse- and RNAse-free water (H2O depc) and deoxynucleotide triphosphates (dNTPs) and MgCl₂.

The PCR amplification process used in the present invention is known to those skilled in the art and is described in detail below.

The method according to the present invention therefore uses an inverse hybridization process on nitrocellulose or nylon strips. In particular, oligonucleotide probes, to which the amplified products are hybridized, are fixed on the said strips. The hybrid product is subsequently detected, preferably by a colorimetric reaction using primers labelled with biotin, i.e. "Mar" is equal to Biotin (Bio), a conjugate, preferably streptavidin conjugated with alkaline phosphatase, and an appropriate coloured substrate.

The probes used in the present invention are preferably: Pos. con. probe: biotinylated oligonucleotide Probe 136: WT 5'- Tgg CAC TTC CCA gCA Tg Probe 136: Mut 5'- TgA CAC TTC CCA gCA Tg Probe 154: WT 5'- CTA TCg TgA AAA CAT gTa CCg Probe 154: Mut 5'- ACT ATC ATg AAA ACA TgT ACC g Probe 171: WT 5'- CCA gTg gAT CAg TAT AgT AAC C Probe 171: Mut-1    5'- CCA gTg gAT Cgg TAT AgT AAC Probe 171: Mut-2     5'- CCA gTg gAT CAT TAT AgT AAC C or their complementary sequences.

The symbol "WT" denotes "wild type", i.e. a normal genotype which shows no mutation.

The symbol "Mut" denotes a mutant genotype; "Mut-1" denotes a mutant genotype of the polymorphism at the 171 codon relating to the replacement of the glutamine amino acid with arginine (Q171R); and "Mut-2" denotes the mutant genotype of the polymorphism at the 171 codon relating to the replacement of the glutamine amino acid with histidine (Q171H).

Preferably, the oligonucleotide probes are fixed on the membrane by enzyme pre-treatment (tailing). Alternatively, according to the invention, the probes are ready for use, or in other words are already fixed on the membrane.

The present invention also relates to a kit for determining predisposition to scrapie, comprising amplification- reagents and detection reagents, wherein the amplification reagents comprise at least one polymerase and an amplification mixture comprising the primers mentioned above, and the detection reagents comprise a denaturing solution, one or more washing solutions, a hybridization solution, a conjugate, a substrate and strips comprising the probes mentioned above.

Preferably, the amplification mixture comprises a buffer, deoxynucleotide triphosphates (dNTPs), the probes mentioned above, MgCl₂ and water.

Preferably, labelling is carried out with biotin and the conjugate of the detection reagents is alkaline streptavidin phosphatase.

### Example 1: Isolation of the DNA

The following reagents were prepared and used in the quantities shown:

| **Reagent** | **Volume** | **Storage** |
|---|---|---|
| Column | 50 | Ambient temp. |
| Collection test tubes (2 ml) | 150 | Ambient temp. |
| Lysis buffer | 12 ml | Ambient temp. |
| Washing buffer 1 (concentrated) | 19 ml | Ambient temp. |
| Washing buffer 2 (concentrated) | 13 ml | Ambient temp. |
| Sterile distilled water | 12 ml | Ambient temp. |
| Protease | 24 mg | Ambient temp. |
| Protease solvent | 1.2 ml | Ambient temp. |

1.2 ml of protease solvent was added to the lyophilized protease. Since diluted protease is stable for 2-3 months if stored at 4°C, it was used even when prepared in advance.

Ethanol was added to washing buffer 1 and washing buffer 2.

The column used was the QIAamp® column made by Quiagen, and the isolation step, described below, was carried out by the method and with the reagents suggested by Quiagen.

The anticoagulant EDTA was added to the whole blood samples. To ensure correct operation, heparin must not be used. 25 µl of sample (whole blood) were pipetted into a sterile 1.5 ml test tube. If the volume of the sample was less than 200 µl, an appropriate volume of PBS was added. If the initial sample was obtained from spots on paper, the blood-spotted piece of paper was diluted in 400 µl of PBS in a sterile 1.5 ml test tube, agitated vigorously for 15 s, and incubated for 10-15 minutes at ambient temperature.

20 µl of protease and 200 µl of lysis buffer were added to the test tube containing the sample treated as above, and the whole was mixed with vigorous agitation for 15 s. The mixture was incubated at 56°C for 10 minutes. 200 µl of ethanol (96-100%) were then added, and the mixture was again agitated vigorously.

The resulting mixture was then pipetted into the column without wetting the upper edge, the cover was closed, and centrifugation was carried out at 8000 r.p.m. for one minute. The column was then placed in a clean collecting test tube, and the test tube containing the filtrate was removed. The column was opened and 500 µl of washing buffer 1 were added. The centrifugation was repeated at 8000 r.p.m. for one minute. The column was then placed in a clean collection test tube, and the test tube containing the filtrate was removed. The column was then opened again and 500 µl of washing buffer 2 were added, and centrifugation was then carried out at maximum speed for 3 minutes. The column was then placed in a 1.5 ml collecting test tube and the test tube containing the filtrate was removed. The DNA was then eluted with 200 µl (50 µl if the initial sample was from a spot on paper) of sterile distilled water at ambient temperature. Incubation was then carried out at ambient temperature for one minute, followed by centrifugation at 8000 r.p.m. for one minute. The DNA sample obtained in this way can be processed directly or frozen (-20°C) until required for use.

Centrifugation was carried out at 8000 r.p.m. for one minute in each case, but higher speeds could also be used, provided that they do not affect the efficiency of the DNA extraction. Centrifugation at lower speeds is also possible, provided that the solutions filter through the column membrane. All the centrifugation was carried out at ambient temperature.

### Example 2: Primer amplification

The following reagents were used for the amplification:
a) amplification mixture:
   - 10X buffer 5 µl/sample
   - MgCl₂ (25 mM) 3 µl/sample
   - dNTP (10 mM) 1 µl/sample
   - Primer 1 (100 pmol/µl) 0.2 µl/sample
      5'-Bio-TgC AgC Tgg AgC AgT ggT Ag
   - Primer 2 (100 pmol/µl) 0.2 µl/sample
      5'-Bio-gTg gTg ACT gTg TgT TgC TTg A
   - H₂O depc 35.4 µl/sample

All the amplification reagents were kept in ice throughout the procedure. All the steps were carried out with the test tubes kept in ice.

A thermocycler test tube was prepared for each sample as prepared in Example 1, and at the same time 44.8 µl of a PCR amplification mixture were prepared from the components listed above and added to the test tube. 5 µl of DNA and 0.2 µl of Tag polymerase (1U) were then added to each test tube. After the thermocycler had been preheated to 94°C, the test tubes were inserted into it and the following amplification programme was run:

| | | |
|---|---|---|
| Initial denaturing | 94°C, 2 mins. | |
| Denaturing | 94°C, 15 s | 35 cycles |
| Annealing | 58°C, 30 s | 35 cycles |
| Extension | 72°C, 30 s | 35 cycles |
| Final extension | 72°C, 3 minutes | |

The resulting amplified samples were kept in ice.

The amplification products obtained could optionally be analysed on 2% agarose gel stained with ethidium bromide. The bands should be visible at 224 bp.

### Example 3: Hybridization and detection

The following components were used for the hybridization and detection step:
a) denaturing solution
b) hybridization solution
c) washing solution A
d) washing solution B
e) conjugate
f) substrate
g) strips

All components were ready for use and their contents were as follows:

| composition of denaturing solution: | |
|---|---|
| NaOH (400 mM) | 16 g/l |
| EDTA | 10 mM |
| Thymolphthalein | 0.1 g/l |
| (dissolved previously in ethanol at a concentration of | |
| 10 mg/ml) | |
| Demineralized H₂O | sufficient to make up 1 litre |

| composition of hybridization solution: | |
|---|---|
| NaCl | 43.83 g/l |
| Sodium citrate | 22.06 g/l |
| N-lauryl sarcosine | 10 g/l |
| SDS | 10 g/l |
| Blocker | 100 ml/l |
| Demineralized H₂O | sufficient to make up 1 litre |

(the blocker was dissolved in maleic acid 16 g/l and NaCl 8.766 g/l pH 7.5 to a final concentration of 10%)

| composition of washing | solution A: |
|---|---|
| Na₂HPO₄x2H₂O | 1.44 g/l |
| KH₂PO₄ | 0.24 g/l |
| NaCl | 2 g/l |
| KCl | 0.05 g/l |
| Tween® 20 | 0.5 ml/l |
| Kathon® | 0.12 ml/l |
| H₂O | sufficient to make up 1 litre |
| pH | 7.4 |

| composition of washing solution B: | |
|---|---|
| Maleic acid | 16 g/l |
| NaCl | 8.766 g/l |
| Tween® 20 | 3 ml/l |
| Kathon® | 3 ml/l |
| H₂O | sufficient to make up 1 litre |
| pH | 7.5 |

### composition of conjugate:

Alkaline streptavidin-phosphatase (the concentration of the enzyme was determined from time to time according to the calibration of the various batches)

| Diluent of the conjugate: | |
|---|---|
| Stabilzime-AP (ready for use) | 1 litre |

### f) composition of the substrate:

4-bromo-4-chloro-3-indolyl phosphate / nitro blue tetrazolium (BCIP/NBT) (ready for use)

### g) composition of the strips:

Whatman membrane
Support (105 x 140 mm piece)
Pos. con. probe: biotinylated oligonucleotide

Probe 136: WT 5'- Tgg CAC TTC CCA gCA Tg

Probe 136: Mut 5'- TgA CAC TTC CCA gCA Tg

Probe 154: WT 5'- CTA TCg TgA AAA CAT gTA CCg

Probe 154: Mut 5'- ACT ATC ATg AAA ACA TgT ACC g

Probe 171: WT 5'- CCA gTg gAT CAg TAT AgT AAC C

Probe 171: Mut-1     5'- CCA gTg gAT Cgg TAT AgT AAC

Probe 171: Mut-2     5'- CCA gTg gAT CAT TAT AgT AAC C

### Preparation of the strips:

### - Preparation for membrane blotting:

### • Probe tailing:

The tailing (poly-T tail) of the probes and of the positive hybridization control was prepared from:

### Tailing mixture:

| 5x buffer | 1x |
|---|---|
| TDT | 0.6U/µl (terminal deoxynucleotidyl |
| | transferase) |
| DTTP | 1.5 nmol/µl (2'deoxythymidine-5'- |
| | triphosphate) |
| H₂O depc | sufficient to make up volume |

The mixture for N probes, plus one, was prepared, after which the sterile test tubes were identified by marking the probe numbers on the stoppers. The mixture was then dispensed into the test tubes and the probes were added to a final dilution of 2 pmol/µl. The test tubes were then incubated at 37°C for 11-12 hours, and on completion of the incubation the reaction was blocked by the addition of EDTA to each test tube to a final concentration of 10 mM. The test tubes were then frozen at -20°C.

### • Blotting:

A washing buffer was prepared by sterile filtration of 0.22 µm of NaCl (52.596 g/l) and sodium citrate (26.469 g/l). A probe diluent (Orange II® , made by Sigma) (3 mg/l) was then added to the washing buffer.

A quantity N of sterile test tubes were then identified, according to the number of probes to be blotted.

The probe diluent buffer (300 µl per test tube x N+1 membranes to be blotted) was then dispensed into the test tubes. The previously prepared probes (probe tailing, as above) were then added to the corresponding test tubes, to the dilution determined for each probe, as follows:
Volumes for blotting:
300 µl of diluent + 0.25 µl of probe 136 WT
300 µl of diluent + 0.5 µl of probe 136 Mut
300 µl of diluent + 1 µl of probe 154 WT
300 µl of diluent + 1 µl of probe 154 Mut
300 µl of diluent + 0.25 µl of probe 171 WT
300 µl of diluent + 0.25 µl of probe 171 Mut-1
300 µl of diluent + 1 µl of probe 171 Mut-2
300 µl of diluent + 1 µl of Pos. Con. probe

The membrane was placed in the manual blotter and, after identification of the channels for the probes and the positive control, 300 µl of probes and positive hybridization control, suitably diluted as indicated above (volumes for blotting), were dispensed. Incubation was carried out for 5 minutes at ambient temperature, the excess liquid being pumped out. Washing was then carried out twice with the washing buffer, the excess liquid being pumped out.

The membrane was then taken from the blotter, placed on the support and dried for two hours at 70°C. 3 mm thick strips of membrane were then cut.

Alternatively, the blotting step can be carried out automatically, using an automatic blotter.

For hybridization and detection, items a) - g) were used as follows:
The water level was controlled in the water bath, and the temperature was controlled at 45°C, a calibrated thermometer being used to ensure that the temperature was in the range 45±0.5°C.

The hybridization solution and washing solution A were preheated to 45°C, ensuring that all the precipitates formed at 4°C were completely dissolved. The DNAT, the conjugate solution, the washing solution B and the colour developer were then left to reach equilibrium at ambient temperature. One strip for each sample was picked up with tweezers and marked with a pencil. 10 µl of DNAT were pipetted into each compartment of the tray (30 µl of DNAT if the initial sample was taken from spots on paper). 10 µl of amplification product were added (30 µl of amplification product if the initial sample was from spots on paper). The solution was mixed with the pipette and remained blue. It was then incubated for 5 minutes at ambient temperature. 1.5 ml of hybridization solution (preheated to 45°C) was then added for each sample and the solution was gently agitated. The blue colour disappeared. One strip for each compartment of the tray was completely immersed. Incubation was then carried out for 30 minutes at 45°C in the water bath with continuous agitation of the strips (50 r.p.m.). At the end of the hybridization, the tray was removed from the bath, the bath being kept at 45°C. The liquid from each compartment was then sucked out with a micropipette or a disposable Pasteur pipette. 1.5 ml of washing solution A (preheated to 45°C) was then added. After approximately 10 seconds, the liquid was removed. 1.5 ml of washing solution A was then added and incubation was carried out for 15 minutes at 45°C in the water bath, with agitation (50 r.p.m.). The liquid was then removed and 1.5 ml of washing solution A was added. After 15 minutes at 45°C in the water bath with agitation (50 r.p.m.), the liquid was removed. All the subsequent incubations were carried out at ambient temperature and with agitation. Preferably, polystyrol sheets were placed between the water in the water bath and the compartments, to insulate the compartments and enable the ambient temperature to be reached more rapidly. 1.5 ml of conjugate solution was then added and incubation was carried out for 15 minutes at ambient temperature with continuous agitation of the strips. After the removal of the liquid, 1.5 ml of washing solution B was added. After approximately 10 seconds, the liquid was removed and 1.5 ml of washing solution B was added. Incubation was then carried out for five minutes at ambient temperature, with continuous agitation of the strips, and the liquid was removed. 1.5 ml of washing solution B was then added and incubation was carried out for 5 minutes at ambient temperature with continuous agitation of the strips. After removal of the liquid, 1 ml of colour developer was added, and incubation was carried out for 15 minutes at ambient temperature in darkness, with continuous agitation. The strips were then washed several times with distilled water or 1 mM EDTA. The strips were then left to dry in the dark on absorbent paper. The bands indicating mutations were then identified.

### Example 4: Identification of mutations

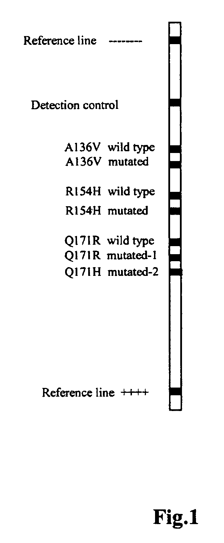

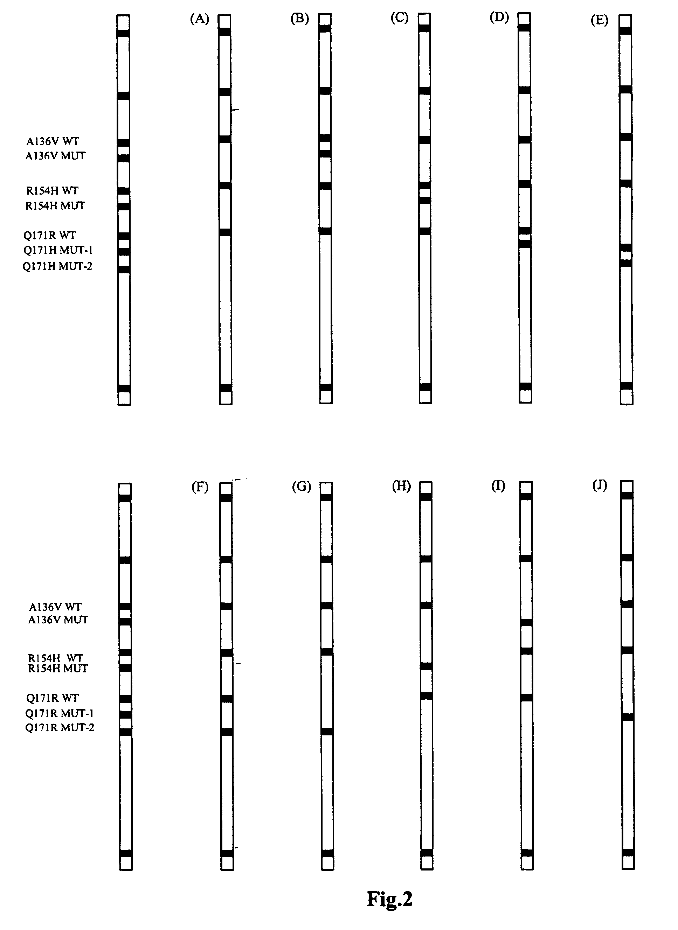

The different strips obtained were identified by the letters A-J as shown in Figure 2. To determine the genotype of a sample, the strips A-J of Figure 2 were compared with the reference strip shown in Fig. 1. The upper line (----) and the lower line (+++++) (marker lines) served as guides for the correct alignment of the sample strip with the reference strip. The intensity of colouring of the different positive bands on each strip could vary, but this had no effect on the interpretation of the results. A positive reaction of the control band (the uppermost band) indicated the correct operation of the conjugate and of the colour developer. This band must always be positive.

For each polymorphic site, one of the following reactivity models could be obtained:

| | |
|---|---|
| 1. only the wild type probe | normal genotype |
| 2. wild type probe and mutant probe | heterozygotic genotype |
| 3. only the mutant probe | mutant homozygotic genotype |

The different strips obtained as shown in Fig. 2 were identified and the results are summarized in the following table:

| | A136V | R154H | Q171H-R | RESULT | |
|---|---|---|---|---|---|
| (A) | norm | norm | norm | normal | |
| (B) | **hetero** | norm | norm | A136V hetero | |
| (C) | norm | **hetero** | **norm** | R154H hetero | |
| (D) | norm | norm | **hetero** | Q171R hetero | |
| (E) | norm | norm | **hetero** | Q171R-H hetero | |
| (F) | norm | norm | hetero | Q171H hetero | |
| (G) | norm | norm | **homo** | Q171H homozygote-2 | mutated |
| (H) | norm | **homo** | norm | R154H homozygotic | mutated |
| (I) | **homo** | norm | norm | A136V homozygotic | mutated |
| (J) | norm | norm | **homo** | Q171R homozygote-1 | mutated |

In order to evaluate the method according to the invention, different known samples were evaluated for each genotype in order to discover whether the genotype was identified both by the conventional extraction method and from blood sampled on absorbent paper. An evaluation was therefore carried out on 75 samples treated by conventional extraction after blood sampling and by spot extraction from a drop of blood sampled on absorbent paper dried and kept for 2-3 days at +4°C.

The following results were obtained:

| **Number of samples evaluated** | **Results obtained by extraction not from spots** | **Results obtained by extraction from spots** |
|---|---|---|
| N.40 normal | N.40 normal | N.40 normal |
| N.20 heterozygotes | N.20 heterozygotes | N.20 heterozygotes |
| N.15 mutated | N.15 mutated | N.15 mutated |

It was found that the genotype was identified perfectly, and that there was perfect matching between conventional and spot extraction.

Clearly, therefore, the method and kit according to the invention are both highly reliable and simple to use. Even low quantities of DNA can therefore be detected with ready-for-use reagents. Furthermore, the method does not entail any particular risks for the operator, and the biological sample can be taken in the field, even by unskilled personnel. The option of using the method with blood drops deposited on paper is particularly useful.

The present invention has been described with reference to the examples and to the figures, but clearly the method and kit according to the present invention can be varied and modified without departing from the scope indicated by the attached claims.

## Claims

1. Method for determining the predisposition of sheep to scrapie by determining the presence or absence of DNA sequences corresponding to one or more polymorphisms at sites 136, 154 and 171 of the prion protein, comprising the following-steps:
a) isolating the DNA from uncoagulated blood;
b) amplifying one or more primers in such a way as to generate amplified products complementary to the said polymorphic sites,
**characterized in that** the primers generating the amplified products of step b) are labelled with one or more labels, and the said labelled amplified products are subsequently hybridized on strips to oligonucleotide probes corresponding to the said polymorphic sites.

2. Method according to Claim 1, wherein the step of isolating the DNA is carried out by column extraction.

3. Method according to Claim 2, wherein the blood sample to be introduced into the column is a sample frozen at -20°C.

4. Method according to Claim 2, wherein the blood sample is a spot of blood on absorbent paper.

5. Method according to any one of the preceding claims, wherein the primers of step b) are:
5'-Mar-TgC AgC Tgg AgC AgT ggT Ag, and
5'-Mar-gTg gTg ACT gTg TgT TgC TTg A

6. Method according to the preceding claims, wherein the oligonucleotide probes are selected from the group consisting of:
Pos. con. probe: biotinylated oligonucleotide Probe 136: WT 5'- Tgg CAC TTC CCA gCA Tg, Probe 136: Mut 5'- TgA CAC TTC CCA gCA Tg, Probe 154: WT 5'- CTA TCg TgA AAA CAT gTA CCg, Probe 154: Mut 5'- ACT ATC ATg AAA ACA TgT ACC g, Probe 171: WT 5'- CCA gTg gAT CAg TAT AgT AAC C, Probe 171: Mut-1    5'- CCA gTg gAT Cgg TAT AgT AAC, and

Probe 171: Mut-2    5'- CCA gTg gAT CAT ATA AgT AAC C

7. Method according to any one of the preceding claims, wherein the oligonucleotide probes are fixed to the membrane by enzyme pre-treatment.

8. Method according to any one of the preceding claims, wherein the hybridization to oligonucleotide probes is detected by a colorimetric reaction.

9. Method according to Claim 8, wherein the colorimetric reaction is obtained by biotin labelling, i.e. Mar is equal to biotin, and streptavidin conjugated with alkaline phosphatase and an appropriate coloured substrate.

10. Kit for determining the predisposition of sheep to scrapie by determining the presence or absence of DNA sequences corresponding to one or more polymorphisms at sites 136, 154 and 171 of the prion protein, comprising amplification reagents and detection reagents, wherein the said amplification reagents comprise at least one polymerase and an amplification mixture comprising the primers 5'-Mar-TgC AgC Tgg AgC AgT ggT Ag, and 5'-Mar-gTg gTg ACT gTg TgT TgC TTg A, and the said detection reagents comprise a denaturing solution, one or more washing solutions, a hybridization solution, a conjugate, a substrate and strips comprising the oligonucleotide probes corresponding to the said polymorphic sites.

11. Kit according to Claim 10, wherein the oligonucleotide probes are selected from the group consisting of:
Pos. con. probe: biotinylated oligonucleotide Probe 136: WT 5'- Tgg CAC TTC CCA gCA Tg, Probe 136: Mut     5'- TgA CAC TTC CCA gCA Tg, Probe 154: WT      5'- CTA TCg TgA AAA CAT gTA CCg, Probe 154: Mut     5'- ACT ATC ATg AAA ACA TgT ACC g, Probe 171: WT 5'- CCA gTg gAT CAg TAT AgT AAC C, Probe 171: Mut-1  5'- CCA gTg gAT Cgg TAT AgT AAC, and Probe 171: Mut-2  5'- CCA gTg gAT CAT TAT AgT AAC C or their complementary sequences.

12. Kit according to Claim 10 or 11, wherein the amplification mixture comprises a buffer, dNTP, MgCl₂ and water.

13. Kit according to any one of Claims 10 to 12, wherein the primers are biotinylated, i.e. Mar is biotin, and the conjugate of the detection reagent is alkaline phosphatase-streptavidin.

14. Kit according to any one of the preceding Claims 10 to 13, wherein the oligonucleotide probes are fixed on the membrane and ready for use.
